# EUROPEAN PATENT APPLICATION

(11) **EP 2 322 858 A1**
(43) Date of publication of application: **18.05.2011**
(21) Application number: 09809923.7
(22) Date of filing: 26.08.2009
(51) Int. Cl.: F24C 1/00, A61L 2/06, A61L 2/24

(54) **COOKING DEVICE**

(30) Priority: 29.08.2008 JP 2008222734
(71) Applicant: Sharp Kabushiki Kaisha, Osaka-shi, Osaka 545-8522 (JP)
(72) Inventor: NISHIURA, Kumiko, 545-8522 , Osaka (JP); TAKAMI, Seiji, 545-8522 , Osaka (JP); SAKANE, Yasuaki, 545-8522 , Osaka (JP)
(74) Representative: Treeby, Philip David William
(86) International application number: PCT/JP2009/064829
(87) International publication number: WO 2010/024272

(57) **Abstract**

A cooking device (1) has a vapor generating device (5) for supplying vapor to a heating chamber (2), a heater (14) for changing saturated water vapor generated by the vapor generating device to superheated water vapor, and a circulation fan (14) for circulating gas in the heating comber (2) through a circulation duct (10). A control device (60) for controlling the entire cooking device (1) performs, according to selection by a user, a bacteria elimination mode such as a resin tableware mode, a ceramic mode, or a kitchenware mode, and the modes have different water vapor temperature settings for different categories of objects from which bacteria are to be eliminated. When a bacteria elimination key (70) provided at an operation section (3b) is pressed, the cooking device (1) enters an operation for selecting among the bacteria elimination modes.

## Description

### Technical Field

The present invention relates to a cooking device that can heat food inside a heating chamber by use of steam.

### Background Art

Household cooking devices that can heat food with steam are increasingly common nowadays. An example is seen in Patent Document 1 listed below.

Steam is also used for sterilization. Examples of devices for sterilization by steam are seen in Patent Documents 2 and 3 listed below. Patent Document 2 discloses a device that sterilizes kitchenware with steam, and Patent Document 3 discloses a device that sterilizes food with steam.

### List of Citations

### Patent Literature

Patent Document 1: JP-2008-25894
Patent Document 2: JP-2004-222816
Patent Document 3: JP-2001-145568

### Summary of Invention

### Technical Problem

Household tableware and kitchenware needs proper sterilization. Different articles, however, have different levels of heat resistance, and should not be sterilized under uniform conditions.

Under the background discussed above, an object of the present invention is to provide a cooking device that can properly sterilize differently heat-resistant articles of tableware and kitchenware used in households.

### Solution to Problem

To achieve the above object, according to the present invention, a cooking device that can heat food inside a heating chamber by use of steam is characterized in that a controller that governs overall control of the cooking device performs a plurality of sterilizing courses with varied steam temperature settings for different sterilization target categories according to a user's selection.

With this configuration, by selecting a sterilization course according to what needs to be sterilized, it is possible to prevent the sterilization target from being hit by steam at an inappropriate temperature.

In the cooking device described above, preferably, when the user presses a sterilization-dedicated key provided in an operation portion, the cooking device enters a mode for selection among the sterilizing courses.

With this configuration, it is possible to select and perform a sterilizing course surely.

### Advantageous Effects of the Invention

According to the present invention, it is possible to properly sterilize differently heat-resistant various articles present in households.

### Brief Description of Drawings

[Fig. 1] is a front sectional view showing a cooking device embodying the invention.
[Fig. 2] is a side sectional view showing the cooking device embodying the invention.
[Fig. 3] is a top sectional view showing the cooking device embodying the invention.
[Fig. 4] is a front sectional view showing the cooking device embodying the invention as cut on a plane through a first exhaust port.
[Fig. 5] is a top sectional view showing the cooking device embodying the invention in a state with an exhaust damper closed.
[Fig. 6] is a top sectional view showing the cooking device embodying the invention in a state with the exhaust damper opened.
[Fig. 7] is a top sectional view showing the cooking device embodying the invention in a state with a feed damper closed.
[Fig. 8] is a top sectional view showing the cooking device embodying the invention in a state with the feed damper opened.
[Fig. 9] is a top sectional view showing the cooking device embodying the invention during cooking by microwaves.
[Fig. 10] is a flow chart showing the operation of the cooking device embodying the invention during cooking by steam.
[Fig. 11] is a top sectional view showing the cooking device embodying the invention during cooling after cooking by steam.
[Fig. 12] is a block configuration diagram of the cooking device embodying the invention.
[Fig. 13] is a front view of the cooking device embodying the invention.
[Fig. 14] is an enlarged view of an operation portion of the cooking device embodying the invention.
[Fig. 15] shows a display screen for selection of a cooking course in the cooking device embodying the invention.
[Fig. 16] shows a display screen for selection of a sterilizing course in the cooking device embodying the invention.
[Fig. 17] is a flow chart of a resin tableware sterilization course in the cooking device embodying the invention.
[Fig. 18] is a flow chart of a china and porcelain tableware sterilization course in the cooking device embodying the invention.
[Fig. 19] is a flow chart of a kitchenware sterilization course in the cooking device embodying the invention.

### Description of Embodiments

An embodiment of the present invention will be described below with reference to the accompanying drawings. A cooking device 1 has, inside a cabinet 4, a heating chamber 2 which is open frontward and openably closed with a door 3. Inside the heating chamber 2, a tray 9 is disposed, and food placed on the tray 9 is accommodated.

Inside the cabinet 4, a circulation duct 10 is provided which runs along the exterior walls of the heating chamber 2. The circulation duct 10 is composed of a side portion 11 at the right side, a top portion 12 at the top, and a side portion 13 at the left side, and these are coupled together in this order. In the side portion 11, approximately at the middle in the depth (front-rear) direction, an evacuation port 10a is formed which is open to the heating chamber 2. In the top portion 12 and the side portion 13, blowout ports 10b and 10c are formed which are open to the heating chamber 2.

In the right-side wall of the heating chamber 2, there are formed, frontward of the evacuation port 10a, a feed port 33 and, rearward of the evacuation port 10a, a first exhaust port 34. The feed port 33 is disposed near the door 3 so that a stream of air blown out via the feed port 33 passes along the door 3. Rearward and downward of the first exhaust port 34, a second exhaust port 35 is formed. The second exhaust port 35 has a smaller opening area than the first exhaust port 34.

In the side portion 11, a circulation fan 14 is disposed which is driven by a circulation motor 14a. As the circulation fan 14 is driven, steam and air inside the heating chamber 2 are sucked into the circulation duct 10 via the evacuation port 10a, and are then blown out via the blowout ports 10b and 10c. In the side portion 11, a temperature sensor 16 is provided which monitors the temperature of the steam and air inside the heating chamber 2 as they enter the side portion 11.

In the top portion 12, a heater 15, such as a sheathed heater, is disposed. The heat radiated from the heater 15 heats the article to be cooked. The heater 15 also heats the steam and air passing through the circulation duct 10, so that the heated steam and air are blown out via the blowout ports 10b and 10c. This keeps the steam and air inside the heating chamber 2 at a predetermined temperature. The steam fed into the heating chamber 2 can also be further heated to produce superheated steam.

On the right side of the heating chamber 2, a removable water tank 7 is disposed. Behind the water tank 7, a steam generator 5 is provided. The steam generator 5 is connected to the water tank 7, and generates steam with heat from a heater (not shown). From the steam generator 5, a steam duct 6 extends to be connected to the side portion 11 of the circulation duct 10. The steam generated in the steam generator 5 passes through the steam duct 6, and enters the side portion 11 of the circulation duct 10 via an inflow port 6a.

Under and on the right side of the heating chamber 2, between the cabinet 4 and the heating chamber 2, an outside air introduction duct 8 is formed. The outside air introduction duct 8 has a suction port 8a formed in the bottom face of the cabinet 4. In a lower part of the outside air introduction duct 8, there are disposed a cooling fan 17, an electric unit 18, and a magnetron 20. In a side part of the outside air introduction duct 8, an air blow duct 30 is disposed. Inside the air blow duct 30, a dilution fan 31 is provided which is driven by a motor 31a.

The electric unit 18 includes driving circuits for driving relevant parts of the cooking device 1 and control circuits for controlling those circuits, and has many components mounted thereon that release heat. The magnetron 20 feeds microwaves into the heating chamber 2 through a wave guide 21. The cooling fan 17 introduces outside air into the outside air introduction duct 8 via the suction port 8a, so as to cool the electric unit 18 and the magnetron 20, which release heat. The outside air introduced into the outside air introduction duct 8 by the cooling fan 17 is drawn by the dilution fan 31. The outside air introduced into the outside air introduction duct 8 eventually passes out of the cabinet 4 via an opening (not shown) formed in the rear face thereof or elsewhere.

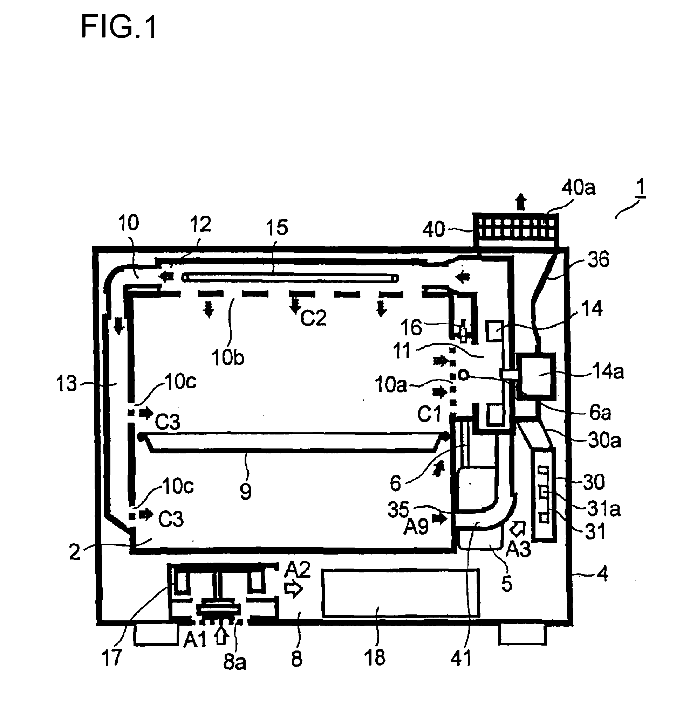

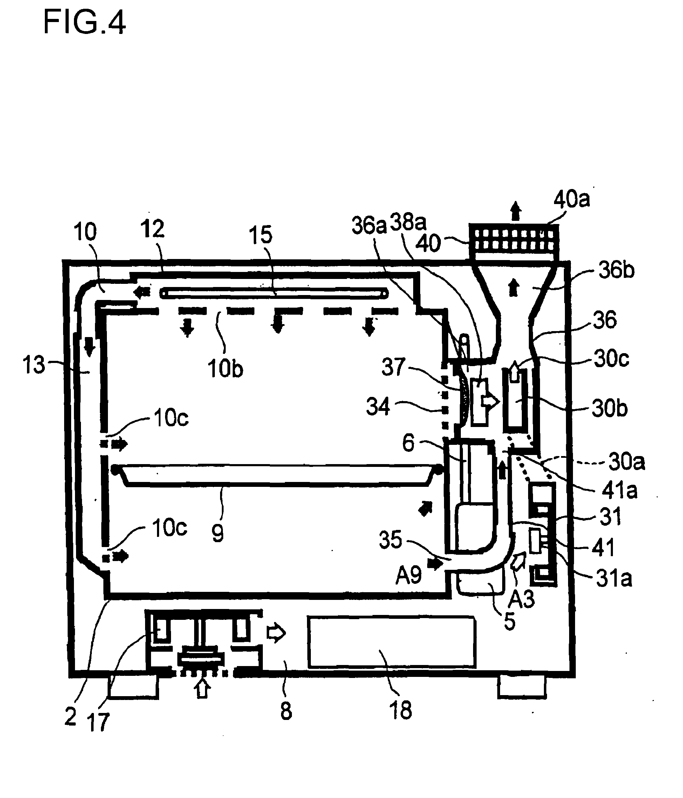

Fig. 4 is a front sectional view of the cooking device 1 as cut on a plane through the first exhaust port 34. In Fig. 1 to 4, at the right-side wall of the heating chamber 2, a first exhaust duct 36 (first exhaust passage) extends from the first exhaust port 34. The first exhaust duct 36 is composed of a transverse passage 36a, which extends horizontally, and an upright passage 36b, which bends upward from the transverse passage 36a. At the upper end of the upright passage 36b, a top cap 40 is provided which is disposed on the top face of the cabinet 4.

In the rear side of the transverse passage 36a, a suction port 38a is formed via which outside air is sucked in through a suction duct 38. In the front side of the transverse passage 36a, a humidity sensor 39 is disposed opposite the suction port 38a. The humidity sensor 39 monitors the humidity of the exhaust air at the first exhaust port 34. Moreover, in the transverse passage 36a, a exhaust damper 37 is provided which selectively opens either the first exhaust port 34 or the suction port 38a.

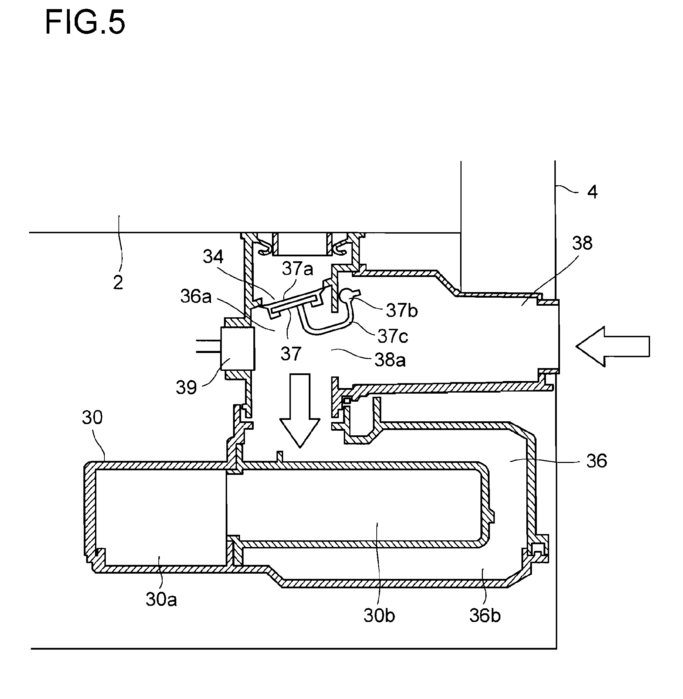

Fig. 5 is a top sectional view showing the details of the exhaust damper 37. The exhaust damper 37 has an arm 37c which is supported at a shaft portion 37b to be pivotable thereabout by a motor (not shown), and at the tip end of the arm 37c, a flexible member 37a is disposed. The arm 37c is a slender solid bar, and is elastically deformable. As shown in Fig. 5, when the flexible member 37a is in close contact with the rim of the first exhaust port 34, the first exhaust port 34 remains closed, and the suction port 38a remains open. In this state, the elasticity of the arm 37c (elastic member) urges the exhaust damper 37 in its closing direction.

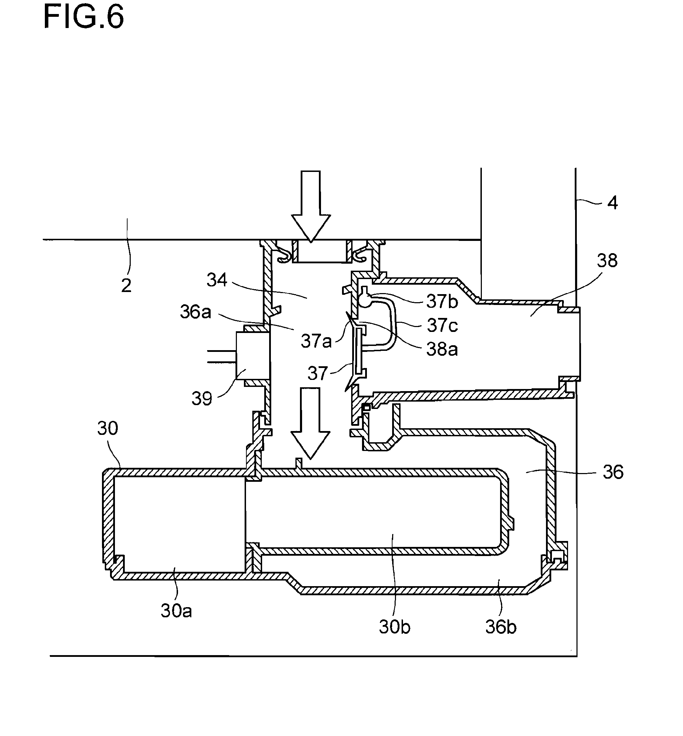

As shown in Fig. 6, when the arm 37c swings so as to bring the flexible member 37a into close contact with the rim of the suction port 38a, the suction port 38a is closed; at this time, the first exhaust port 34 is opened. Thus, the exhaust damper 37 serves as a suction damper that opens and closes the suction port 38a. Opening and closing the first exhaust port 34 and the suction port 38a with a single exhaust damper 37 helps reduce the number of components.

The upright passage 36b of the first exhaust duct 36 is, in an upper part thereof, given an increased passage cross-sectional area and coupled to the top cap 40. The top cap 40 is, at the free end thereof, open frontward to form a discharge port 40a. The discharge port 40a is disposed with the lower end thereof away from the top face of the cabinet 4. The aim is to prevent entry of water into the first exhaust duct 36 in case water is spilt on top of the cabinet 4.

The upper and lower walls of the top cap 40 are inclined 20° or more upward relative to the horizontal line. Thus, the exhaust air discharged out via the discharge port 40a of the top cap 40 is blown out obliquely upward, at 20° or more relative to the horizontal line. Locating the lower end of the discharge port 40a away from the top face of the cabinet 4 and discharging the exhaust air obliquely upward via the discharge port 40a helps reduce steam that passes along the top face of the cabinet 4. This reduces condensation on the top face of the cabinet 4.

At the lower end of the discharge port 40a, a projection (not shown) may be provided that projects frontward. This helps cancel the Coanda effect, whereby steam from a lower part of the discharge port 40a passes along the top face of the cabinet 4. This further reduces condensation on the top face of the cabinet 4. Forming the tip end of the projection in an acute angle is further preferable, because that further cancels the Coanda effect.

To the lower face of the transverse passage 36a of the first exhaust duct 36, a second exhaust duct 41 (second exhaust passage) extending from the second exhaust port 35 is coupled at a coupling 41a. Thus, the humidity sensor 39 disposed in the transverse passage 36a is disposed upstream of the coupling 41a provided in the upright passage 36b. The second exhaust duct 41 may be formed of flexible tube.

The second exhaust duct 41 is formed to have a smaller passage cross-sectional area than the first exhaust duct 36. The exhaust air via the second exhaust port 35 passes through the second exhaust duct 41, enters the first exhaust duct via the coupling 41a, and is discharged out via the discharge port 40a of the top cap 40. The lower face of the first exhaust duct 36 is inclined downward toward the coupling 41a.

In a lower part of the air blow duct 30 at the side of the heating chamber 2, the dilution fan 31 is disposed, and in an upper part of the air blow duct 30, an air blow passage of the dilution fan 31 is formed. The air blow duct 30 has an upright passage 30a, a transverse passage 30b, and a nozzle portion 30c. The upright passage 30a extends upward from the dilution fan 31. The transverse passage 30b bends rearward from the upright passage 30a, and is disposed into the first exhaust duct 36.

The nozzle portion 30c further bends upward from the transverse passage 30b, and has, at the end thereof, an opening 30d that is open upward. Thus, inside the first exhaust duct 36, an ejector is formed, so that, as the dilution fan 31 is driven, a stream of air is produced that runs from the first exhaust port 34 to the open end (discharge port 40a). Here, the coupling 41a and the suction port 38a are disposed upstream of the opening 30d. Thus, a negative pressure is applied to the second exhaust duct 41 and the suction duct 38, and this prevents a backward stream of air.

In the transverse passage 30b, a lowered portion 30g is formed whose level is lower than the lower end of the part of the transverse passage 30b connecting to the upright passage 30a. At one end of the lowered portion 30g, a sub-nozzle portion 30e is formed which is open to the top cap 40. The sub-nozzle portion 30e has the lower wall thereof inclined upward. Thus, as the dilution fan 31 is driven, the stream of air that passes from the sub-nozzle portion 30e into the first exhaust duct 36 runs upward, and this prevents a backward stream of air into the second exhaust duct 41.

The lower wall of the lowered portion 30g is inclined downward toward the sub-nozzle portion 30e. Thus, even in case water is spilt on the top face of the cabinet 4 and enters the air blow duct 30 via the top cap 40, the water is collected in the lowered portion 30g and is drained into the first exhaust duct 36via the sub-nozzle portion 30e. The water that has entered the first exhaust duct 36 runs down the inclined lower face, and is collected, through the second exhaust duct 41, in the heating chamber 2. In this way, the motor 31a of the dilution fan 31 is prevented from being soaked in water.

The air blow duct 30 has, at the part thereof where the upright passage 30a connects to the transverse passage 30b, a rib 30f that extends upward. The rib 30f is disposed inside the transverse passage 30b, and lopsided toward the heating chamber 2. The motor 31a of the dilution fan 31 is disposed inside the upright passage 30a, and lopsided toward the heating chamber 2. That is, the rib 30f is disposed lopsided in the same direction as the motor 31a. Thus, in case water is spilt on the top face of the cabinet 4 and enters the air blow duct 30, the motor 31a of the dilution fan 31 is more surely prevented from being soaked in water.

In an upper part of the upright passage 30a of the air blow duct 30, a feed tube 32 is provided to branch off. The feed tube 32 is connected to a feed duct 50 which extends from the feed port 33 of the heating chamber 2. The feed tube 32 and the feed duct 50 form an air feed passage through which, as the dilution fan 31 is driven, air is fed into the heating chamber 2 via the feed port 33. The feed tube 32 may be formed as a duct.

The feed duct 50 has a leak hole 50a formed therein opposite the feed port 33, and is provided with a feed damper 51 which selectively opens, or closes, either the feed port 33 or the leak hole 50a. The feed duct 50 forms the housing for the feed damper 51.

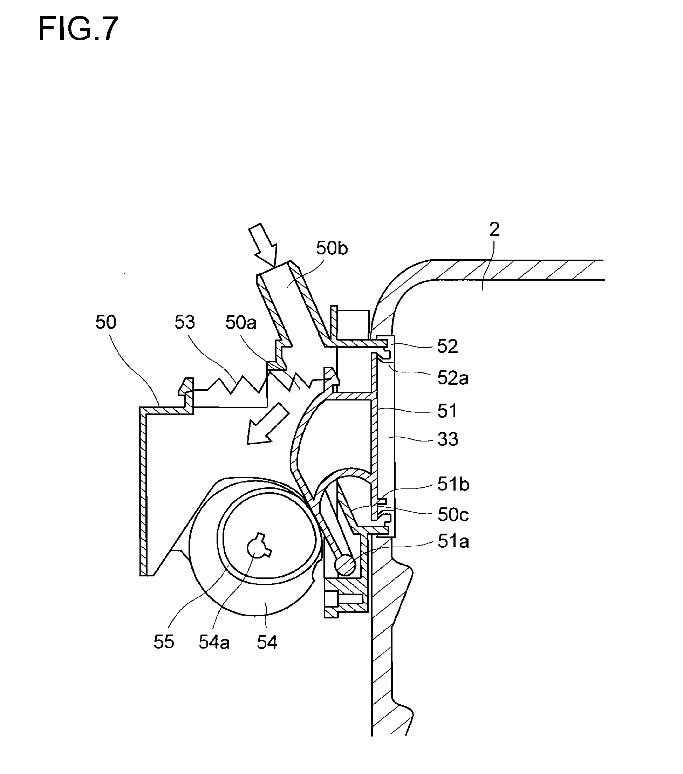

Fig. 7 is a side sectional view showing the details of the feed duct 50 and the feed damper 51. The feed duct 50, which forms the housing for the feed damper 51, has, in a side face thereof, fitted with a ring-shaped gasket 52 formed as a flexible member, and is fitted in the feed port 33. This keeps air-tightness between the feed port 33 and the feed duct 50.

The gasket 52 has a ring-shaped projection 52a formed around the inner circumference thereof. As shown in Fig. 7, when closed, the feed damper 51 is in close contact with the projection 52a so that no stream of air leaks through the feed port 33. Thus, the gasket 52 that keeps air-tightness between the feed port 33 and the feed duct 50 keeps air-tightness between the feed port 33 and the feed damper 51, and this helps reduce the number of components.

The feed damper 51 is supported at a shaft portion 51a at the lower end thereof to be pivotable thereabout, and is urged in its opening direction by a tension spring 53 which is coupled to the feed duct 50. Behind the feed damper 51, a motor 54 is disposed. The shaft 54a of the motor 54 is fitted with a cam 55 which makes contact with the rear face of the feed damper 51.

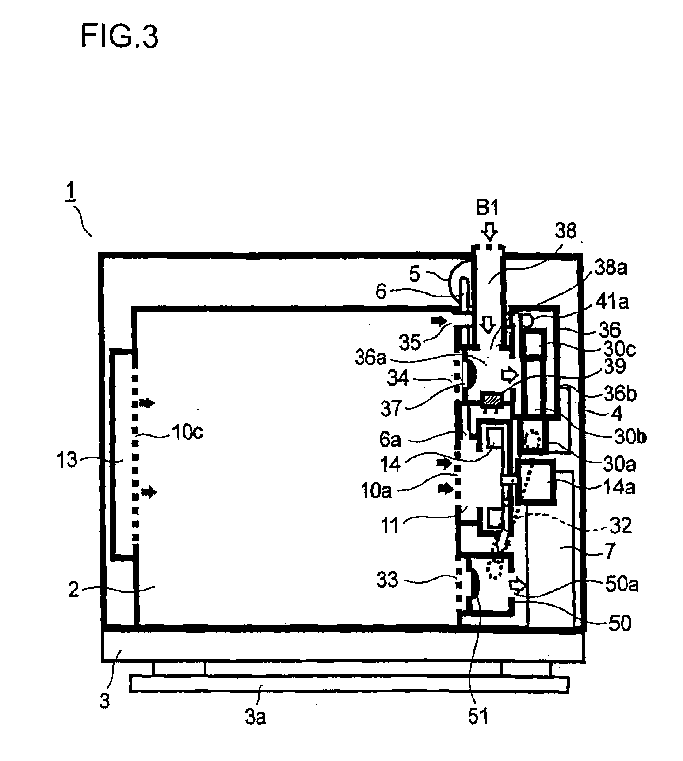

In an upper part of the feed duct 50, an inflow portion 50b is formed to which the feed tube 32 is connected. The inflow portion 50b is so inclined as to be increasingly low frontward, and through the inflow portion 50b, a stream of air is blown out via the feed port 33 toward the door 3 (see Fig. 3). The leak hole 50a is provided under the inflow portion 50b. The wall face 50c around the leak hole 50a is inclined relative to the vertical line.

As the motor 54 is driven, the feed damper 51 is pressed by the cam 55 so that, against the urging force of the tension spring 53, the feed damper 51 is brought into close contact with the projection 52a of the gasket 52. This allows the feed damper 51 to be kept closed by the pressing force of the cam 55 which is formed as an inelastic member. At this time, the leak hole 50a is opened. As the dilution fan 30 is driven, a stream of air that enters the feed duct 50 through the inflow portion 50b returns into the outside air introduction duct 8 via the leak hole 50a.

The exhaust damper 37 is urged in its closing direction by the arm 37c (see Fig. 5), which is an elastic member, and the feed damper 51 is kept closed by the cam 55, which is an inelastic member. Thus, when the exhaust damper 37 and the feed damper 51 are closed, if the pressure inside the heating chamber 2 becomes abnormally high, the exhaust damper 37 opens against the urging force of the arm 37c to exhaust air. This helps increase the safety of the cooking device 1, and also prevents a backward stream of steam via the feed port 33.

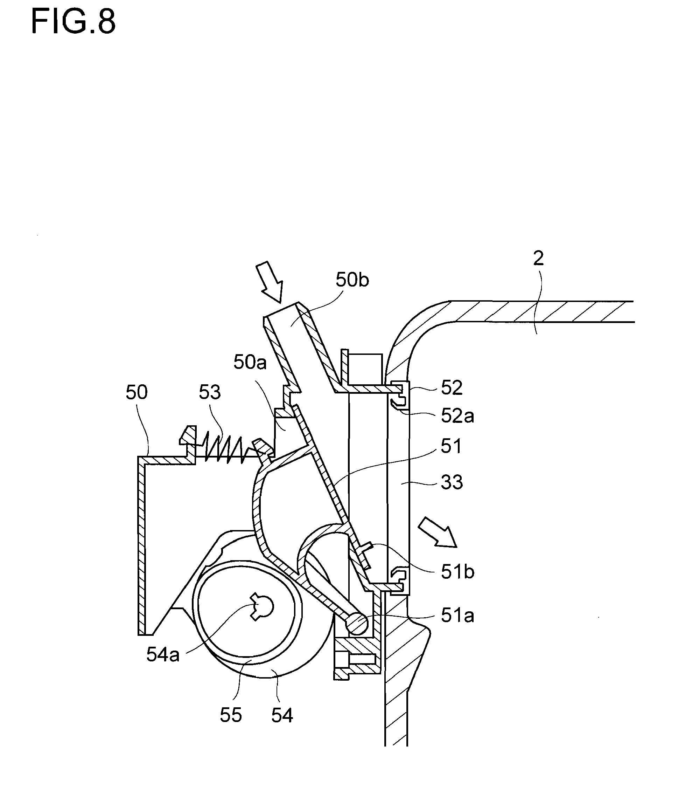

As shown in Fig. 8, when the cam 55 rotates in the direction retreating from the feed damper 51, the urging force of the tension spring 53 opens the feed damper 51. The feed damper 51 is thus kept in contact with the inclined wall face 50c and hence open. At this time, the leak hole 50a is closed. Now, as the air dilution fan 30 is driven, a stream of air that enters the feed duct 50 through the inflow portion 50b is fed into the heating chamber 2 via the feed port 33.

On a lower part of the feed damper 51, a rib serving as a gutter 51b is formed to project toward the heating chamber 2. The gutter 51b is formed in the shape of a cornered C open at the top and toward the heating chamber 2. When opened, the feed damper 51 is exposed to the hot gas inside the heating chamber 2, and this causes condensation to form on its surface. The feed damper 51, then resting on the wall face 50c and inclined, lets condensed water run down to collect in the gutter 51b. When the feed damper 51 is closed, the condensed water is collected from the gutter 51b into the heating chamber 2. This prevents water from leaking into the outside air introduction duct 8 (see Fig. 1) in which the electric unit 18 is disposed.

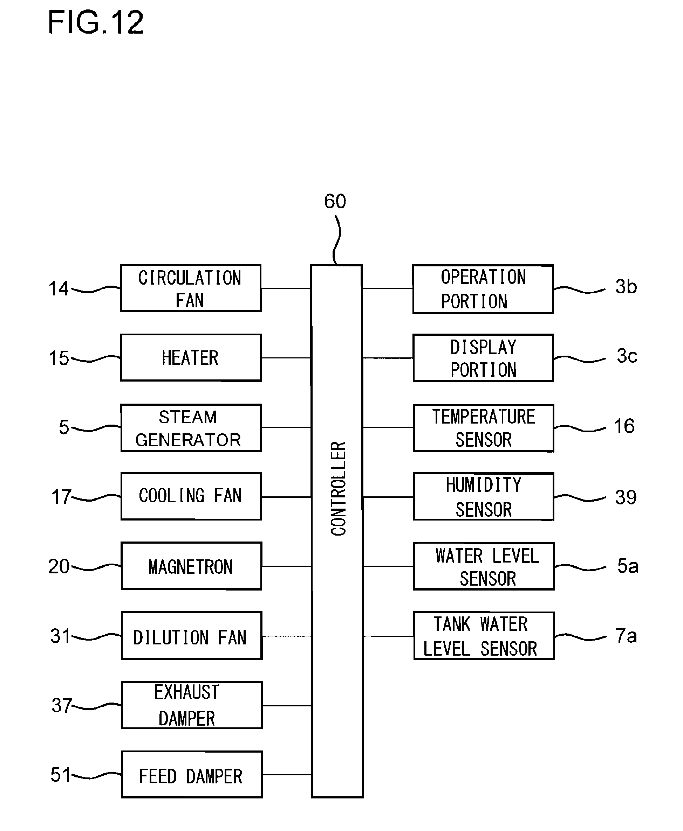

The elements involved in the control of the cooking device 1 are shown in Fig. 12. Overall control is governed by a controller 60. Connected to the controller 60 are the circulation fan 14, the heater 15, the steam generator 5, the cooling fan 17, the magnetron 20, the dilution fan 31, the exhaust damper 37, the feed damper 51, the temperature sensor 16, and the humidity sensor 39, which have been mentioned above; additionally connected to the package can 60 are an operation portion 3b, a display portion 3c, a water level sensor 5a, and a tank water level sensor 7a. The operation portion 3b is provided on the front face of the door 3, and includes means of operation such as push buttons, a dial, etc. The display portion 3d is provided within the operation portion 3b, and includes a means of display such as a liquid crystal display panel. The water level sensor 5a is provided in the steam generator 5 to monitor the level of the water inside it; the tank water level sensor 7a is provided in the water tank 7 to monitor the level of water inside it.

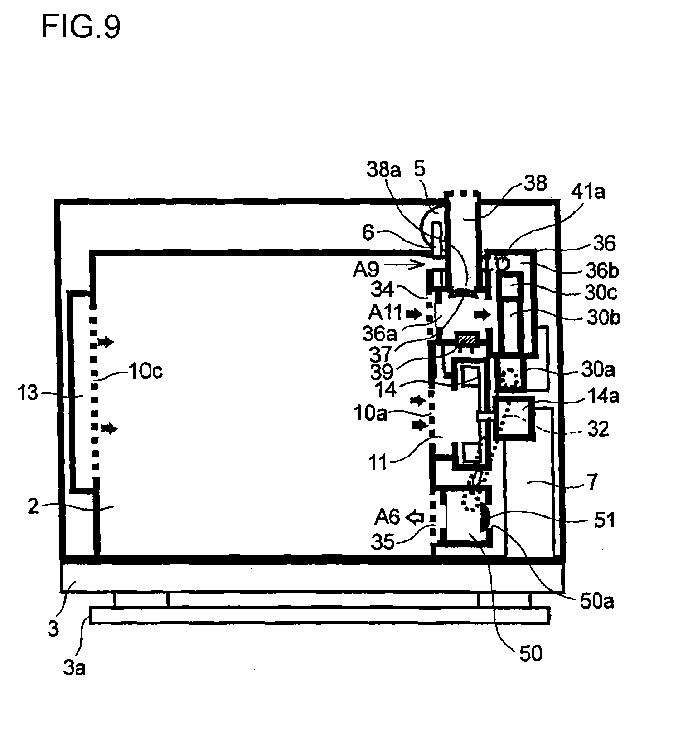

In the cooking device 1 configured as described above, when cooking by microwaves is started, the magnetron 20 is driven. Moreover, as shown in Fig. 9, the feed damper 51 and the exhaust damper 37 open the feed port 33 and the first exhaust port 34, and the cooling fan 17 and the dilution fan 31 are driven. The magnetron 20 feeds microwaves into the heating chamber 2 through the wave guide 21, and an article to be cooked is heated by microwaves.

The cooling fan 17 introduces outside air into the outside air introduction duct 8 via the suction port 8a as indicated by arrow A1 (see Fig. 1). The outside air that has entered the outside air introduction duct 8 cools the electric unit 18 and the magnetron 20 as indicated by arrow A2 (see Fig. 1). The outside air that has cooled the electric unit 18 and the magnetron 20 and has thereby been heated is drawn by the dilution fan 31 as indicated by arrow A3 (see Fig. 1).

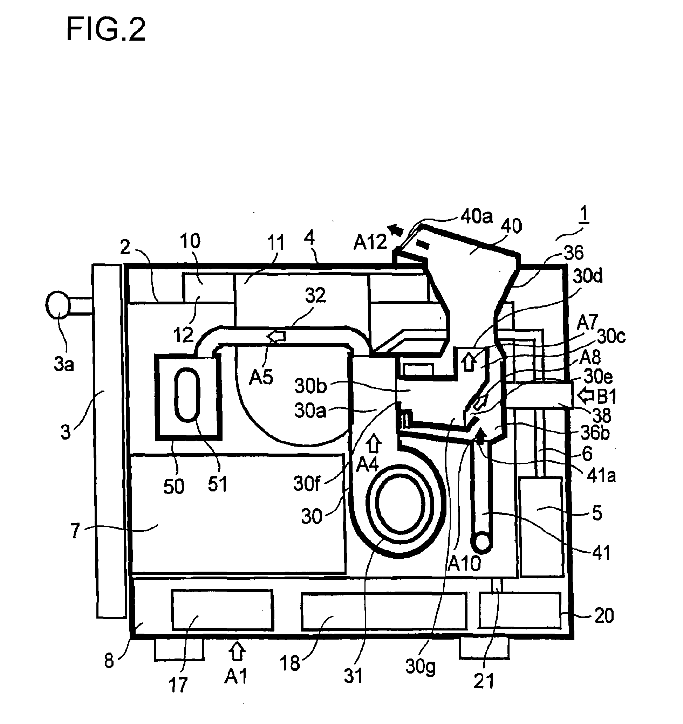

The dilution fan 31 sends the outside air out, so that the outside air passes through the air blow duct 30, the feed tube 32, and the feed duct 50 as indicated by arrows A4 and A5 (see Fig. 2). The outside air that has entered the feed duct 50 is fed into the heating chamber 2 via the feed port 33 as indicated by arrow A6 (see Fig. 9).

Here, the stream of air blown out via the feed port 33 disposed near the door 3 passes along the door 3. Thus, the air heated as a result of cooling the electric unit 18 and the magnetron 20 prevents condensation on the door 3. Moreover, the inflow portion 50b of the feed duct 50 blows the stream of air out toward the door 3. This ensures that the stream of air blown out via the feed port 33 reaches the door 3, and thereby helps further prevent condensation.

In addition, as indicated by arrows A7 and A8 (see Fig. 2), the outside air is fed into the first exhaust duct 36 via the nozzle portion 30c and the sub-nozzle portion 30e of the air blow duct 30.

The air inside the heating chamber 2 is exhausted via the first and second exhaust ports 34 and 35 as indicated by arrows A9 and A11 (see Fig. 9). The exhaust air via the second exhaust port 35 passes through the second exhaust duct 41, and enters the first exhaust duct 36 via the coupling 41a as indicated by arrow A10 (see Fig. 2).

The exhaust air via the first exhaust port 34 makes contact with the humidity sensor 39 in the transverse passage 36a of the first exhaust duct 36. Thus, the humidity inside the heating chamber 2 is detected. The exhaust air through the transverse passage 36a passes through the upright passage 36b to join the exhaust air through the second exhaust duct 41 and goes up, so as to be discharged out via the discharge port 40a of the top cap 40 as indicated by arrow A12 (see Fig. 2). Here, since the nozzle portion 30c and the sub-nozzle portion 30e of the air blow duct 30 form an ejector, a negative pressure is applied to the second exhaust duct 41 and the feed port 33. This prevents a backward stream of exhaust air.

Heated by microwaves, the article being cooked gives off steam. When the humidity inside the heating chamber 2 becomes equal to a predetermined level, the humidity sensor 39 detects it, and thereby the time to end cooking is recognized. Thus, cooking by microwaves is ended.

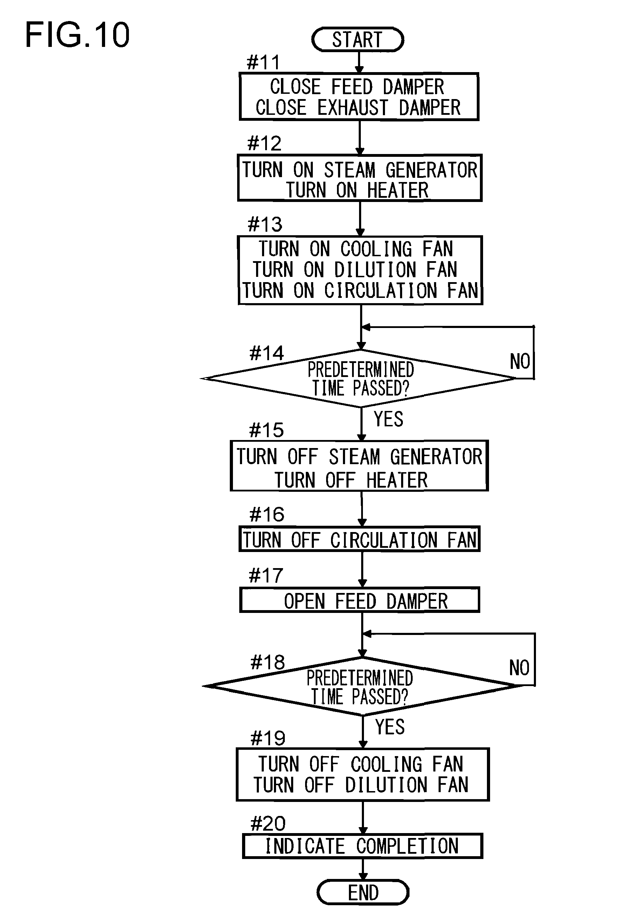

Fig. 10 is a flow chart showing how cooking by steam proceeds. When cooking by steam is started, at step #11, as shown in Fig. 3 described previously, the feed damper 51 and the exhaust damper 37 close the feed port 33 and the first exhaust port 34. At step #12, the steam generator 5 and the heater 15 are driven. As a result, steam is fed into the circulation duct 10 and is heated by the heater 15 to produce superheated steam.

At step #13, the cooling fan 17, the dilution fan 31, and the circulation fan 14 are driven. In a similar manner as described previously, as the cooling fan 17 and the dilution fan 31 are driven, outside air enters the outside air introduction duct 8 via the suction port 8a. The outside air is then fed into the first exhaust duct 36 via the nozzle portion 30c and sub-nozzle portion 30e of the air blow duct 30.

As the circulation fan 14 is driven, the steam inside the heating chamber 2 enters the circulation duct 10 via the suction port 8a as indicated by arrow C1 (see Fig. 1). The steam that has entered the circulation duct 10 is blown out into the heating chamber 2 via the blowout ports 10b and 10c as indicated by arrows C2 and C3 (see Fig. 1). As a result, the steam inside the heating chamber 2 circulates through the circulation duct 10. The steam passing through the circulation duct 10 is heated by the heater 15, so that the steam is kept at a required temperature to perform cooking. The temperature and driving duration of the heater 15 may be adjusted so as to perform cooking with saturated steam.

Feeding steam from the steam generator 5 into the heating chamber 2 causes steam to pass out of the heating chamber 2 via the second exhaust port 35 as indicated by arrow A9 (see Fig 1). This keeps the pressure inside the heating chamber 2 constant. The second exhaust duct 41 has a smaller passage cross-sectional area than the first exhaust duct 36, and thus allows less steam to pass out. This contributes to increased heating efficiency.

The exhaust air via the second exhaust port 35 passes through the second exhaust duct 41, and enters the first exhaust duct 36 via the coupling 41a. Since the dilution fan 31 feeds outside air into the first exhaust duct 36, the exhaust via the second exhaust port 35 is diluted before being discharged out. This allows steam to be discharged after being cooled down, and thus helps increase the safety of the cooking device 1.

At this time, the outside air passing through the outside air introduction duct 8 exchanges heat with the electric unit 18 and the magnetron 20 and is thereby heated. Thus, the exhaust air via the second exhaust port 35 is mixed with heated outside air, and this lowers the relative humidity of the exhaust air. It is thus possible to reduce condensation inside the first and second exhaust ducts 36 and 41.

Disposing the humidity sensor 39 upstream of the coupling 41a of the second exhaust duct 41 helps reduce contact between the steam passing from the second exhaust duct 41 into the first exhaust duct 36 and the humidity sensor 39. This helps reduce condensation on the humidity sensor 39, and makes it possible to perform cooking by microwaves satisfactorily the next time.

As a result of outside air entering the first exhaust duct 36 via the nozzle portion 30c and the sub-nozzle portion 30e, a negative pressure produced by an ejector is applied to the suction duct 38. As a result, as indicated by arrow B1 (see Figs. 2 and 3), outside air is sucked into the first exhaust duct 36 via the suction port 38a. This helps further dilute the exhaust air via the second exhaust port 35. In addition, since the humidity sensor 39 is disposed between the suction port 38a and the coupling 41a, the humidity sensor 39 makes contact with the outside air from the suction port 38a. This allows the humidity sensor 39 to be dried, and helps further prevent condensation on the humidity sensor 39.

The negative pressure produced by the ejector is also applied to the second exhaust duct 41, and this prevents a backward stream through the second exhaust duct 41. Since the second exhaust duct 41 has a small passage cross-sectional area, when condensation forms, the second exhaust duct 41 may become closed airtight, causing the pressure inside the heating chamber 2 to rise instead of being sucked up by the negative pressure by the ejector. To avoid this, it is preferable to drive the dilution fan 31 in an intermittent operation in which driven periods and halted periods alternate. In this way, while the dilution fan 31 is halted, the condensation inside the second exhaust duct 41 runs down and collects in the heating chamber 2, and this helps maintain the pressure inside the heating chamber 2.

At step #14, passage of a predetermined cooking time is waited for. When the predetermined time passes and cooking ends, then, at step #15, the steam generator 5 and the heater 15 are stopped. At step #16, the circulation fan 14 is stopped.

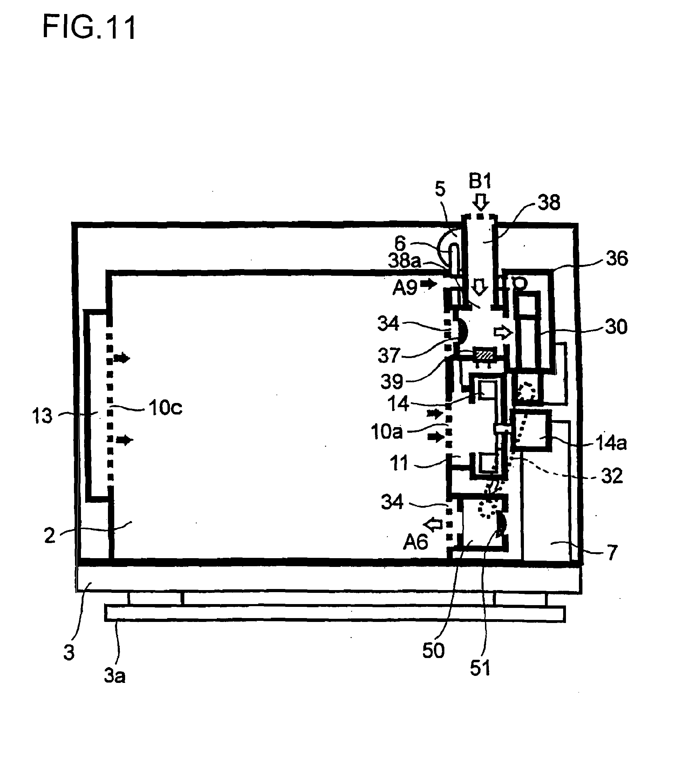

At step #17, as shown in Fig. 11, the feed damper 51 is opened. Now, outside air is fed through the feed tube 32 and the air blow duct 30 into the heating chamber 2 via the feed port 33 (arrow A6), and is exhausted via the second exhaust port 35. As a result, the inside of the heating chamber 2 is cooled. At this time, the exhaust damper 37 remains closed, and this prevents contact between the steam inside the heating chamber 2 and the humidity sensor 39.

At step #18, passage of a predetermined cooling time is waited for. When the predetermined time passes, an advance is made to step #19. It is also possible to monitor the temperature inside the heating chamber 2 so that, when it reaches a predetermined temperature, an advance is made to step #19. At step #19, the cooling fan 17 and the dilution fan 31 are stopped. At step #20, completion of cooking is indicated.

It is also possible to stop the feeding of steam while driving the heater 15 and the circulation fan 14 to perform cooking with hot air. In that case also, through operation similar to cooking by steam, cooking is achieved. Since no steam is used then, the exhaust damper 37 may be opened during cooling after cooking. This increases the exhaustion rate, and thus allows quick cooling of the inside of the heating chamber 2.

It is also possible to open, during cooling, the feed damper 51 that has been closed during cooking by steam or hot air and, a predetermined period thereafter, open the exhaust damper 37. This makes it possible to first cool to a certain degree by exhausting a small amount of gas via the second exhaust port 35 and then further cool by exhausting a large amount of gas via the first exhaust port 34. In this way, it is possible to secure safety and achieve quick cooling.

Instead of cooling the heating chamber 2 by opening, after cooking, the feed damper 51 that has been closed during cooking by steam or hot air, it is also possible to open the feed damper 51 a predetermined period (for example, one minute) before completion of cooling. This permits the heating chamber 2 to have been cooled, and hence the door 3 to be opened, on completion of cooling, and thus makes the cooking device 1 more convenient to use.

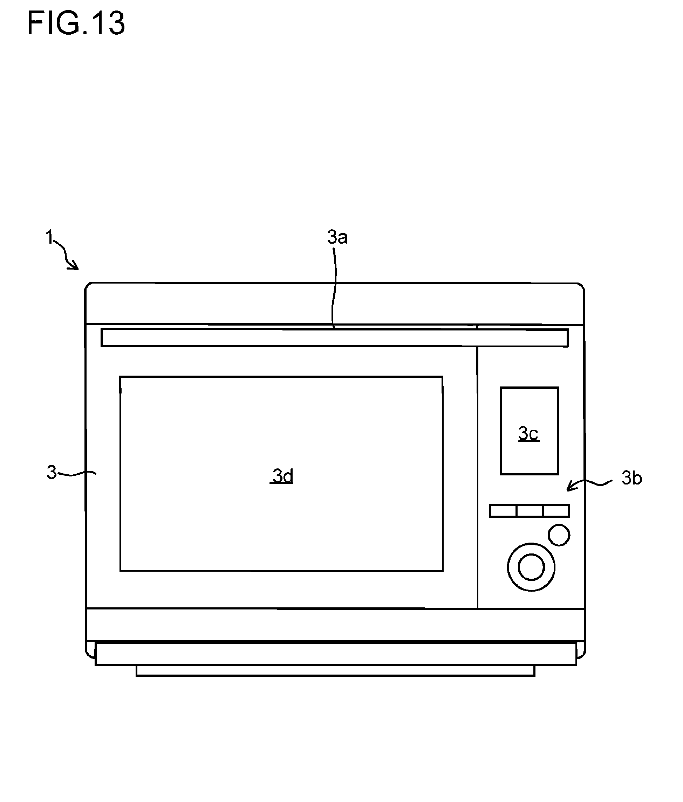

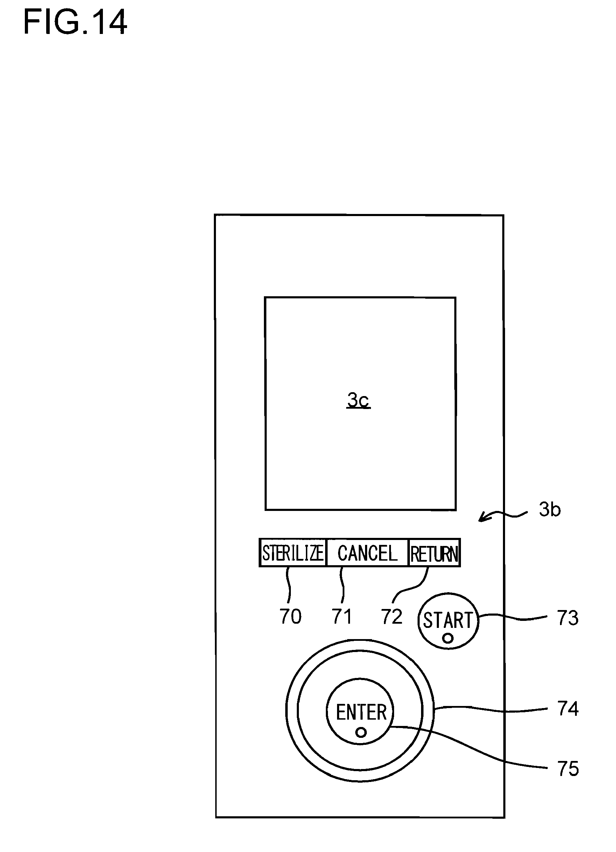

The cooking device 1 can sterilize articles put inside it by use of steam. The mechanism will now be described with reference to Figs. 13 to 19.

The door 3 is provided with, in an upper part, a handle 3a and, in a right-side part, an operation portion 3b. The operation portion 3b includes a display portion 3c. On the left side of the operation portion 3b, a sight window 3d is provided which allows visual inspection inside the heating chamber 2. The sight window 3d has a pane of heat-resistant glass set therein.

The display portion 3c is built around a liquid crystal display panel. Under the display portion 3c, three rectangular keys are arranged in a horizontal row. These are, from left, a "sterilize" key 70, a "cancel" key 71, and a "return" key 72. The "sterilize" key 70 is a key dedicated to sterilization, and pressing it invokes a mode for selection among a plurality of sterilizing courses. The "cancel" key 71 is used to cancel an operation. The "return" key 72 is used to return to the stage one operation before.

Closely under the "return" key 72, a circular "start" key 73 is provided. On the lower left side of the "start" key 73, that is, right under the "cancel" key 71, a selection dial 74 is provided. At the center of the selection dial 74, an "enter" key 75 is provided. While the selection dial 74 is rotary, the "enter" key 75 is not.

The "start" key 73 is used to turn the power on and off, and to start the selected course. The selection dial 74 is used to select among different courses and different preferences displayed on the display portion 3c. The "enter" key 75 is used to fix a selection.

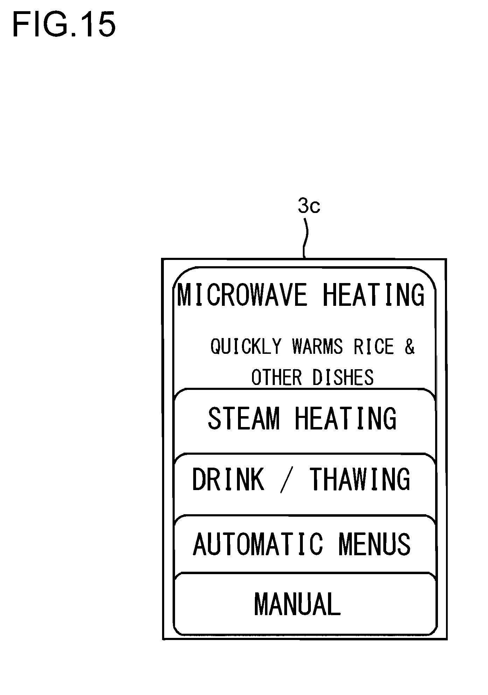

During ordinary cooking, displayed on the display portion 3c are, as shown in Fig. 15, tabs for different cooking courses, such as "microwave cooking," "steam cooking," "drink warming/thawing," "automatic menus," and "manual." When the user turns the selection dial 74 and selects one of the tabs, the selected tab becomes bigger than the other, and a message showing an outline of that cooking course is displayed. When the user confirms his selection correct and presses the "enter" key 75, a screen for selection of preferences to be specified for execution of the cooking course is displayed. When the user makes necessary selections and presses the "start" key 73, the cooking course is executed.

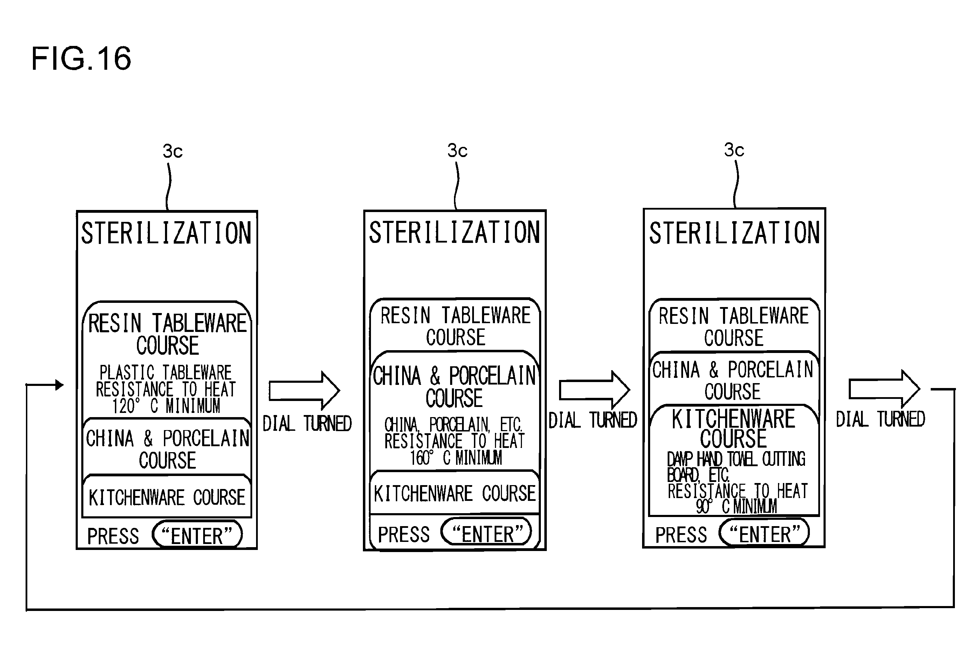

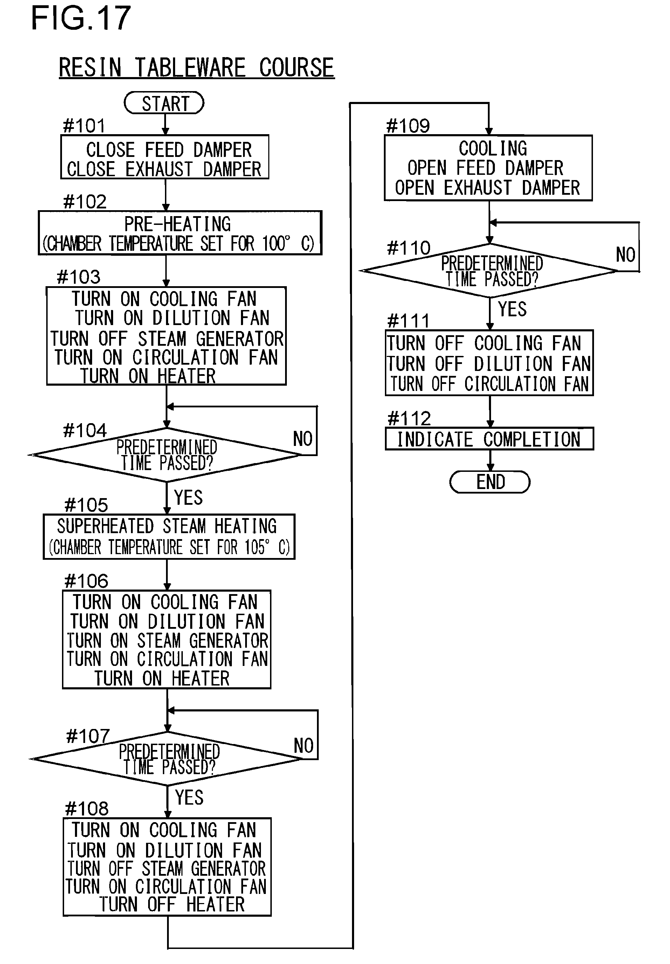

Pressing the "sterilize" key 70 invokes a mode for selection among a plurality of sterilizing courses, and the screen on the display portion 3c switches to a sterilizing course selection screen as shown in Fig. 16. Displayed on the sterilizing course selection screen are tabs for three sterilizing courses, namely "resin tableware course," "china and porcelain course," and "kitchenware course." When the user turns the selection dial 74 and selects one of the tabs, the selected tab becomes bigger than the other, and a message showing an outline of that sterilizing course is displayed. When the user confirms his selection correct and presses the "enter" key 75, a screen for selection of preferences to be specified for execution of the sterilization course is displayed. When the user makes necessary selections and presses the "start" key 73, the sterilization course is executed. Now, with reference to Figs. 17 to 19, how each sterilizing course proceeds will be described.

[Resin Tableware Course] The procedure shown in a flow chart in Fig. 17 starts when the user puts a sterilization target - suppose here, for example, a piece of plastic tableware heat-resistant to 120 °C (degrees Celsius) or more - in the heating chamber 2 and presses the "start" key 73. At step #101, the feed damper 51 and the exhaust damper 37 are closed. At step #102, a pre-heating process is started. In the resin tableware course, the temperature of the steam used for sterilization is low; therefore, if the heating chamber 2 is cold, condensation forms on the interior surface of the heating chamber 2. To prevent this, the heating chamber is pre-heated to about a temperature at which condensation does not form. The temperature inside the heating chamber during pre-heating is set for 100 °C.

At step #103, the circulation fan 14 and the heater 15 are turned on, so that the heating chamber 2 is pre-heated. The cooling fan 17 and the dilution fan 31 are also turned on. At step #104, the time that has passed since the pre-heating process was started is checked so that, if a predetermined time (t13) has passed, an advance is made to step #105.

At step #105, a superheated steam heating process is started. The temperature inside the heating chamber 2 is set for 105 °C. At step #106, the steam generator 5, the circulation fan 14, and the heater 15 are all turned on, so that superheated steam is blown out into the heating chamber 2, and the superheated steam inside the heating chamber 2 circulates. The cooling fan 17 and the dilution fan 31 are also turned on.

At step #107, the time (t23) that has passed since the superheated steam heating process was started is checked so that, if a predetermined time has passed, an advance is made to step #108. At step #108, the steam generator 5 and the heater 15 are turned off, but the circulation fan 14 is kept on, so that the gas inside the heating chamber 2 continues to circulate. The cooling fan 17 and the dilution fan 31 are also kept on.

At step #109, a cooling process is started. At this time, the feed damper 51 and the exhaust damper 37 are opened. The hot gas inside the heating chamber 2 is exhausted through the first exhaust duct 36 out via the discharge port 40a.

At step #110, the time (t33) that has passed since the cooling process was started is checked so that, if a predetermined time has passed, an advance is made to step #111. At step #111, the circulation fan 14 is turned off. The cooling fan 17 and the dilution fan 31 are also turned off. Then, an advance is made to step #112, where completion of the process is indicated. Completion of the process is indicated visibly on the display portion 3c and audibly, with an audible signal, from a sound generator (not shown). This ends the resin tableware sterilizing course.

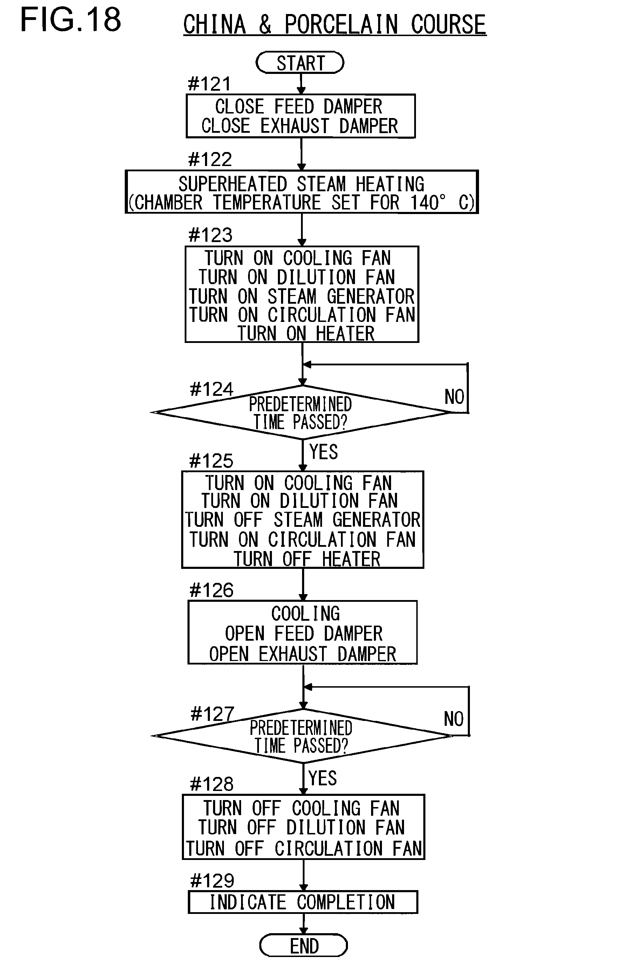

[China and Porcelain Course] The procedure shown in a flow chart in Fig. 18 starts when the user puts a sterilization target - suppose here, for example, a china or porcelain heat-resistant to 160 °C or more - in the heating chamber 2 and presses the "start" key 73. At step #121, the feed damper 51 and the exhaust damper 37 are closed. At step #122, a superheated steam heating process is started. In the china and porcelain course, the steam used for sterilization is superheated steam at a high temperature; therefore, no condensation forms even without pre-heating. Accordingly, no pre-heating process is involved here.

In the superheated steam heating process, the temperature inside the heating chamber 2 is set for 140 °C. At step #123, the steam generator 5, the circulation fan 14, and the heater 15 are all turned on, so that superheated steam is blown out into the heating chamber 2, and the superheated steam inside the heating chamber 2 circulates. The cooling fan 17 and the dilution fan 31 are also turned on.

At step #124, the time (t21) that has passed since the superheated steam heating process was started is checked so that, if a predetermined time has passed, an advance is made to step #125. At step #125, the steam generator 5 and the heater 15 are turned off, but the circulation fan 14 is kept on, so that the gas inside the heating chamber 2 continues to circulate. The cooling fan 17 and the dilution fan 31 are also kept on.

At step #126, a cooling process is started. At this time, the feed damper 51 and the exhaust damper 37 are opened. The hot gas inside the heating chamber 2 is exhausted through the first exhaust duct 36 out via the discharge port 40a.

At step #127, the time (t31) that has passed since the cooling process was started is checked so that, if a predetermined time has passed, an advance is made to step #128. At step #128, the circulation fan 14 is turned off. The cooling fan 17 and the dilution fan 31 are also turned off. Then, an advance is made to step #129, where completion of the process is indicated. Completion of the process is indicated visibly on the display portion 3c and audibly, with an audible signal, from a sound generator (not shown). This ends the china and porcelain sterilizing course.

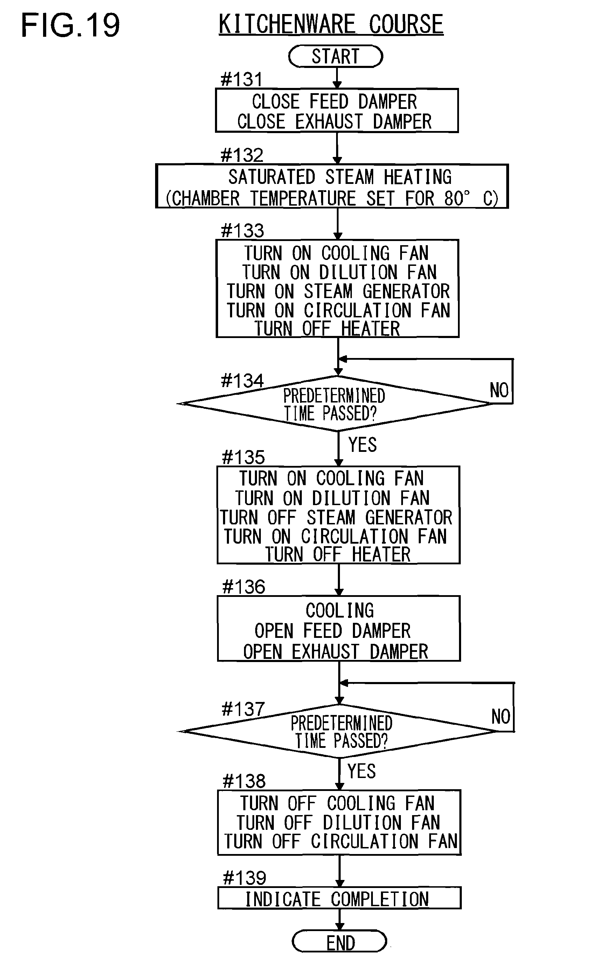

[Kitchenware Course] The procedure shown in a flow chart in Fig. 19 starts when the user puts a sterilization target - suppose here, for example, a damp hand towel or a cutting board heat-resistant to 90 °C or more - in the heating chamber 2 and presses the "start" key 73. At step #131, the feed damper 51 and the exhaust damper 37 are closed. At step #132, a saturated steam heating process is started. In the kitchenware course, the steam used for sterilization is saturated steam; accordingly, condensation on the heating chamber 2 and the sterilization target is taken as a matter of course, and no pre-heating process is involved here.

In the saturated steam heating process, the temperature inside the heating chamber 2 is set for 80 °C. At step #133, the steam generator 5 and the circulation fan 14 are turned on, whereas the heater 15 is turned off, so that saturated steam is blown out into the heating chamber 2, and the saturated steam inside the heating chamber 2 circulates. The cooling fan 17 and the dilution fan 31 are also turned on.

At step #134, the time (t12) that has passed since the superheated steam heating process was started is checked so that, if a predetermined time has passed, an advance is made to step #135. At step #135, in addition to the heater 15, the steam generator 5 is turned off, but the circulation fan 14 is kept on, so that the gas inside the heating chamber 2 continues to circulate. The cooling fan 17 and the dilution fan 31 are also kept on.

At step #136, a cooling process is started. At this time, the feed damper 51 and the exhaust damper 37 are opened. The hot gas inside the heating chamber 2 is exhausted through the first exhaust duct 36 out via the discharge port 40a.

At step #137, the time (t22) that has passed since the cooling process was started is checked so that, if a predetermined time has passed, an advance is made to step #138. At step #138, the circulation fan 14 is turned off. The cooling fan 17 and the dilution fan 31 are also turned off. Then, an advance is made to step #139, where completion of the process is indicated. Completion of the process is indicated visibly on the display portion 3c and audibly, with an audible signal, from a sound generator (not shown). This ends the kitchenware sterilizing course.

In this way, by selecting a sterilizing course according to what needs to be sterilized, it is possible to prevent the sterilization target from being hit by steam at an inappropriate temperature.

The embodiment described specifically above is in no way meant to limit the scope of the present invention; the invention may be carried out with many variations and modifications made without departing from the spirit of the invention. For example, it is possible to provide a course for sterilizing foods as well as those for sterilizing tableware and kitchenware.

### Industrial Applicability

The present invention finds wide application in cooking devices provided with a function of heating food inside a heating chamber by use of steam.

### List of Reference Signs

- 1: cooking device
- 2: heating chamber
- 3: door
- 3b: operation portion
- 3c: display portion
- 5: steam generator
- 9: tray
- 10: circulation duct
- 14: circulation fan
- 15: heater
- 16: temperature sensor
- 17: cooling fan
- 18: electric unit
- 20: magnetron
- 31: dilution fan
- 37: exhaust damper
- 39: humidity sensor
- 51: feed damper
- 60: controller
- 70: "sterilize" key
- 74: selection dial
- 75: "enter" key

## Claims

1. A cooking device that can heat food inside a heating chamber by use of steam, **characterized in that**
a controller that governs overall control of the cooking device performs a plurality of sterilizing courses with varied steam temperature settings for different sterilization target categories according to a user's selection.

2. The cooking device according to claim 1, **characterized in that**
when the user presses a sterilization-dedicated key provided in an operation portion, the cooking device enters a mode for selection among the sterilizing courses.
